# EUROPEAN PATENT SPECIFICATION

(11) **EP 2 436 667 B1**
(45) Date of publication and mention of the grant of the patent: **31.07.2013**
(21) Application number: 10460036.6
(22) Date of filing: 08.09.2010
(51) Int. Cl.: C07C 67/44, C07C 69/28

(54) **A method to manufacture a mixture of aliphatic hydroxyesters, especially from isobutyric aldehyde**
Verfahren zur Herstellung einer Mischung aus aliphatischen Hydroxyestern, insbesondere aus Isobutyraldehyd
Procédé de fabrication d'un mélange d'hydroxyesters aliphatiques, en particulier d'aldéhyde isobutyrique

(43) Date of publication of application: 04.04.2012
(73) Proprietor: Politechnika Opolska, 45-271 Opole (PL); Zak, Spolka Akcyjna, 47-220 Kedzierzyn-Kozle (PL)
(72) Inventor: Tic, Wilhelm Jan, 45-720 Opole (PL); Guzialowska-Tic, Joanna, 45-720 Opole (PL); Sutor, Eugeniusz, 47-232 Kedzierzyn-Kozle (PL); Bulanda, Wojciech, 47-230 Kedzierzyn-Kozle (PL)
(74) Representative: Surmiak, Wieslawa Halina

(56) References cited:
- DE-A1- 10 207 747
- US-A- 5 180 847
- DATABASE CA [Online] CHEMICAL ABSTRACTS SERVICE, COLUMBUS, OHIO, US; TIC, WILHELM JAN ET AL: "Manufacture of 2,2,4-trimethyl-1,3-pentanediol monoisobutyrate", XP002623270, retrieved from STN Database accession no. 2008:697768 & PL 194 619 B1 (INSTYTUT CIEZKIEJ SYNTEZY ORGANICZNEJ BLACHOWNIA, POL.; ZAKLADY AZOTOW) 29 June 2007 (2007-06-29)

## Description

The present invention relates to a method to manufacture, especially from isobutyric aldehyde, a mixture of aliphatic hydroxyesters which is a mixture of two isomeric forms: 3-hydroxy-2,2,4-trimethylpentyl isobutyrate and 3-hydroxy-2,2-dimethyl-1-(1-methylethyl)propyl isobutyrate occurring in an equimolar ratio and which mixture is intended as a coalescent solvent in the manufacturing of water-based paints and varnishes.

Methods to manufacture aliphatic hydroxyesters, from aldehydes which contain α hydrogen, in a condensation reaction are known in the art. The reaction follows the Tishchenko mechanism of aldol condensation, during which condensation of 2 molecules of isobutyric aldehyde with the formation of an aldol in the form of 3-hydroxy-2,2,4-trimethylpentanal takes place in the first step whereupon, in the second step of condensation and disproportioning of another molecule of isobutyric aldehyde and aldol, 2,2,4-trimethyl-1,3-pentanediol isobutyrate is formed. The resulting hydroxyester occurs in the form of two isomers which are in equilibrium.

In the method to obtain 2,2,4-trimethyl-1,3-pentanediol isobutyrate according to the patent EP 0186076 isobutyric aldehyde and an alkaline catalyst solution are added, at the same time, into a tubular reactor with packing by means of a dip leg and the reaction is conducted at temperatures in the range 50-80°C for 0.25-2 hr. Aqueous sodium hydroxide solution at a concentration by weight of 5-30% is used as catalyst.

The method to obtain 2,2,4-trimethyl-1,3-pentanediol monoisobutyrate or a mixture containing 2,2,4-trimetyl-1,3-pentanediol monoisobutyrate from isobutyric aldehyde for use as an additive to paints and varnishes was disclosed in the patent DE 10207747. The reaction is carried out in two steps in the presence of a basic catalyst. The condensation reaction is conducted at temperatures in the range 60-130°C in the first step, and 140-250°C in the second step. Selectivities of approx. 90% relative to the esters and yields of a maximum of 57% were obtained.

According to the method disclosed in the US Patent 5180847, derivatives of 2,2,4-trimethyl-1,3-pentanediol are obtained from isobutyric aldehyde. The process to manufacture 2,2,4-trimethyl-1,3-pentanediol monoisobutyrate includes three steps. In the first step, condensation of isobutyric aldehyde in the presence of an alkaline metal hydroxide takes place and a reaction mixture comprising 2,2,4-trimethyl-1,3-pentanediol isobutyrate and alkaline metal isobutyrate are obtained. In the second step, an aqueous phase containing alkaline metal isobutyrate is separated from the reaction mixture and, in the third step, water is removed from the aqueous phase until a concentration of approx. 12.5% by weight of sodium isobutyrate is obtained whereupon a concentrated sodium isobutyrate is added to the first reaction step. The introduction of sodium isobutyrate reduces water concentration in the reaction medium, whereby higher conversions and higher yields are obtained.

According to the method to obtain 2,2,4-trimethyl-1,3-pentanediol monoisobutyrate, as disclosed in the patent PL 194619, the isobutyric aldehyde condensation reaction is carried out in two steps. In the first step, a tubular reactor is filled with a premixed solution of isobutyric aldehyde with a catalyst in the form of an aqueous sodium hydroxide solution at a concentration in the range 30-60%, and the isobutyric aldehyde-to-sodium hydroxide ratio by mole is 0.02-0.06. The reaction is conducted at temperatures in the range 50-80°C. The resulting mixture is added to a tank-type reactor equipped with a distillation column to distill any unreacted isobutyric aldehyde in its top section. Any unreacted raw materials are further processed at temperatures in the range 60-100°C during 30-300 sec at a temperature in the range 64-80°C at the top of the column.

Disclosed in the patent RU 2082712 is a method for the separation of 2,2,4-trimethyl-1,3-pentanediol monoisobutyrate from condensation products in the presence of an aqueous alkaline solution as catalyst by rectification with the use of a separating agent in the form of 2,2,4-trimethyl-1,3-pentanediol diisobutyrate. Rectification is conducted in a column at pressures in the range 67-200 kPa and a temperature at the bottom of the column in the range 150-190°C with separation of isobutyric aldehyde at the top of the column, whereupon a spent liquid from the first column is rectified in the second column at pressures in the range 0.7-13,3 kPa and temperatures in the range 150-250°C at the bottom of the column, and then the spent liquid is sent to a third column with product being collected from top of the column. An ester with a purity of 95% is separated at a yield of 75%.

Methods to manufacture a mixture of aliphatic hydroxyesters from isobutyric aldehyde which are known in the art, although they do enable the desired product to be obtained, are characterized by selectivities usually not higher than 90% and purities up to 95%, whereby their applications in industrial chemistry are much limited.

The gist of the method of the invention for manufacturing a mixture of aliphatic hydroxyesters, especially from isobutyric aldehyde, is that the isobutyric aldehyde condensation reaction is carried out in the presence of a mixture of sodium hydroxide and sodium isobutyrate as catalyst in a weight ratio of 3:1, first at 58-60% conversions of isobutyric aldehyde to 2,2,4-trimethyl-1,3-pentanediol isobutyrate at temperatures in the range 58-62°C during 8-12 minutes and then at 68-72% conversions of isobutyric aldehyde to 2,2,4-trimethyl-1,3-pentanediol isobutyrate at temperatures in the range 88-92°C during 3-7 minutes. Then, from a mixture of 3-hydroxy-2,2,4-trimethylpentyl isobutyrate and 3-hydroxy-2,2-dimethyl-1-(1-methylethyl)propyl isobutyrate, an aqueous solution of the catalyst is separated by allowing to stand and elution from the organic phase of the mixture after the condensation reaction. Distillation of the remaining portion of the mixture, after the condensation reaction, is carried out in four steps. In the first step, distillation is carried out in a column with three structural packing zones at an atmospheric pressure at a temperature of 61 °C at the top of the column and a temperature of 100°C at the bottom of the column, at a reflux-to-distillate ratio by weight of 0.5:1, while process water in the amount of 0.1 % is used as a separating agent added to a stream of the mixture introduced to the first column, and isobutyric aldehyde is collected at the top of it. In the second step, distillation is carried out in a column with two structural packing beds, at a vacuum of 120 kPa abs., at a temperature of 49°C at the top of the column and a temperature of 140°C at the bottom of the column, at a reflux-to-distillate ratio by weight of 3:1, while the aqueous ester-containing raw mixture, which is a spent liquid from the first column, is subjected to distillation to distill off any water therefrom. In the third step, distillation is carried out in a column with two packing beds, at a vacuum of 1.3 kPa abs., at a temperature of 121°C at the top of the column and a temperature of 138°C at bottom of the column, at a reflux-to-distillate ratio by weight of 15:1, and light ends containing 2,2,4-trimethylpentanediol-1,3 are collected at the top of the column. In the fourth step, distillation is carried out in a column with two packing beds, at a vacuum of 1.3 kPa abs., at a temperature of 126°C at the top of the column and a temperature of 145°C at the bottom of the column, at a reflux-to-distillate ratio by weight of 1:1, and a mixture of 3-hydroxy-2,2,4-trimethylpentyl isobutyrate and 3-hydroxy-2,2-dimethyl-1-(1-methylethyl)propyl isobutyrate is collected at the top of the column.

The method of the invention for manufacturing a mixture of aliphatic hydroxyesters, especially from isobutyric aldehyde, does not only provide high selectivities of the products obtained but also a very high purity of the mixture of two isomeric forms: 3-hydroxy-2,2,4-trimethylpentyl isobutyrate and 3-hydroxy-2,2-dimethyl-1-(1-methylethyl)propyl isobutyrate occurring in an equimolar ratio, which enables the mixture to be categorized among volatile organic compounds for use as solvents and coalescents for paints and varnishes.

### Example (related)

A mixer equipped with a nitrogen blanket is filled in a continuous manner with 7.58 kg/hr of fresh isobutyric aldehyde and with regenerated isobutyric aldehyde after its separation from the mixture after the condensation reaction at the rate of 2.54 kg/hr. The resulting mixture is added in a continuous manner to the first reactor while also filling the reactor with 48% fresh soda lye at the rate of 0.31 kg/hr and with an aqueous catalyst solution, after its separation from the mixture after the condensation reaction, at the rate of 0.41 kg/hr. The condensation reaction is carried out at temperatures in the range 60°C, at a nitrogen blanket pressure of approx. 0.015 kPa, for 10 minutes. The reaction mixture is then introduced to the second flow reactor in which the isobutyric aldehyde condensation reaction is carried out at a temperature of 90°C for 10 minutes at a nitrogen blanket pressure of approx. 0.015 kPa. The post-reaction mixture is then sent to a phase separator. Conversion of isobutyric aldehyde in the first reactor is approx. 59% while reaction selectivity to the mixture of isomers of 2,2,4-trimethyl-1,3-pentanediol isobutyrate is 97%, total conversion after the second reactor is 69% and total selectivity is 95%. After being cooled in the cooler the mixture after the condensation reaction is sent, in a continuous manner at the rate of 10.84 kg/hr, to a decanter where part of the catalyst solution is preliminarily separated and recycled to the sodium hydroxide solution preparation unit. After preliminary separation of the aqueous phase which contains the catalyst, the organic phase is sent to a tank-type reactor in which it is mechanically mixed with water at a ratio of 9:1 by weight. Separation of the organic phase, which contains a mixture of condensation reaction products along with any unreacted isobutyric aldehyde, from the aqueous phase which contains sodium isobutyrate and sodium hydroxide, is conducted by centrifugation in a centrifuge at a temperature of 40°C. A raw mixture after the condensation reaction and catalyst removal, which contains an unreacted isobutyric aldehyde in the amount of approx. 21%, is subjected to distillation to distill off any aldehyde in a column which is packed with three beds of Mellapak 350.Y structural packing from Sulzer. The distillation step is carried out at an atmospheric pressure and at a temperature of 61 °C at the top of the column and 100°C at the bottom of the column and at a reflux-to-distillate ratio by weight of 0.5:1. Process water, which is a separating agent that enables the total separation of isobutyric aldehyde from the mixture of esters, is fed into the lower section of the column from a buffer tank at the rate of 0.1 kg/hr. A distillate stream, containing isobutyric aldehyde, is recycled in a continuous manner to the condensation process. A distillation residue in the form of a raw ester, containing approx. 2.5% water, is added to a vacuum rectification column packed with two Mellapak 250.Y packing beds where light components, mainly water, are evaporated at a reduced absolute pressure of 12 kPa abs. and at a temperature of 49°C at the top of the column and a temperature of 140°C at the bottom of the column and at a reflux-to-distillate ratio of 3:1. Any non-evaporated raw esters contain light ends in the form of 2,2,4-trimethylpentanediol-1,3 in the amount of approx. 3%. The light ends are removed in a column which is packed with two beds of Gauze BX packing from Sulzer and filled with raw esters from the dewatering column evaporator. Any vapors from the top of the column are condensed in the condenser and sent to the storage tank. Part of the distillate is recycled as a reflux. An ester fraction without any light ends is collected at the bottom and is sent by means of a pump to the next column for removal of heavy ends. The process in the column and the auxiliary devices is conducted at a vacuum of 1.3 kPa abs. and at a temperature of 121 °C at the top of the column and a temperature of 138°C at the bottom of the column and at a reflux-to-distillate ratio by weight of 15:1. Separation of the heavy ends, i.e., approx. 1.1 % 2,2,4-trimethyl-1,3-pentanediol diisobutyrate, from 2,2,4-trimethyl-1,3-pentanediol isobutyrate takes place in a vacuum column which is packed with two beds of Mellapak 350.Y structural packing. A mixture of isomers 2,2,4-trimethyl-1,3-pentanediol isobutyrate is added at the top of the column and, after being condensed in the condenser, is collected at the product tank. The process in the vacuum column and its auxiliary devices is conducted at a vacuum of 1.3 kPa abs. at a temperature of 126°C at the top of the column and a temperature of 145°C at the bottom of the column and at a reflux-to-distillate ratio by weight of 1:1.

According to the method of the invention for manufacturing a mixture of aliphatic hydroxyesters, especially from isobutyric aldehyde, a product containing 99% of a mixture of two isomeric forms: 3-hydroxy-2,2,4-trimethylpentyl isobutyrate and 3-hydroxy-2,2-dimethyl-1-(1-methylethyl)propyl isobutyrate is obtained.

## Claims

1. A method to manufacture a mixture of aliphatic hydroxyesters, especially from isobutyric aldehyde, by condensation of isobutyric aldehyde in the presence of a basic catalyst which was partly recovered from the process, followed by distillation to separate any unreacted isobutyric aldehyde and evaporation of 2,2,4-trimethyl-1,3-pentanediol and distillation to separate 2,2,4-trimethyl-1,3-pentanediol isobutyrate wherein the isobutyric aldehyde condensation reaction is carried out in the presence of a mixture of sodium hydroxide and sodium isobutyrate as catalyst in a weight ratio of 3:1, first at 58-60% conversions of isobutyric aldehyde to 2,2,4-trimethyl-1,3-pentanediol isobutyrate at temperatures in the range 58-62°C during 8-12 minutes, and then at 68-72% conversions of isobutyric aldehyde to 2,2,4-trimethyl-1,3-pentanediol isobutyrate at temperatures in the range 88-92°C during 3-7 minutes whereupon, from a mixture of 3-hydroxy-2,2,4-trimethylpentyl isobutyrate and 3-hydroxy-2,2-dimethyl-1-(1-methylethyl)propyl isobutyrate, an aqueous solution of the catalyst is separated by allowing to stand and elution from the organic phase of the mixture after the condensation reaction while the process of distillation of the remaining portion of the mixture after the condensation reaction is carried out in four steps so that, in the first step, distillation is carried out in a column with three structural packing zones at an atmospheric pressure, at a temperature of 61 °C at the top of the column and a temperature of 100°C at the bottom of the column, at a reflux-to-distillate ratio by weight of 0.5:1, while process water in the amount of 0.1% is used as a separating agent and is added to a stream of the mixture introduced to the first column and isobutyric aldehyde is collected at the top of it, in the second step, distillation is carried out in a column with two structural packing beds at a vacuum of 12 kPa abs., at a temperature of 49°C at the top of the column and at a temperature of 140°C at bottom of the column, at a reflux-to-distillate ratio by weight of 3:1 while the aqueous ester-containing raw mixture, which is a spent liquid from the first column, is subjected to distillation to distill off any water therefrom, in the third step, distillation is carried out in a column with two packing beds, at a vacuum of 1.3 kPa abs., at a temperature of 121°C at the top of the column and a temperature of 138°C at the bottom of the column, at a reflux-to-distillate ratio by weight of 15:1, and light ends containing 2,2,4-trimethylpentanediol-1,3 are collected at the top of the column, and in the fourth step distillation is carried out in a column with two packing beds, at a vacuum of 1.3 kPa abs., at a temperature of 126°C at the top of the column and a temperature of 145°C at the bottom of the column and at a reflux-to-distillate ratio by weight of 1:1.

## Patentansprüche

1. Verfahren zur Herstellung einer Mischung aus aliphatischen Hydroxyestem, insbesondere aus Isobutyraldehyd, bestehend in Kondensationsreaktion von Isobutyraldehyd in Gegenwart eines alkalischen Katalysator zum Teil aus dem Prozess zurückgewonnenen und dann Abtrennen des nicht umgesetzten Isobutyralaldehydes durch Destillation und Verdampfung des 2,2,4-Trimethyl-1 ,3-Pentandiol, und destillative Abtrennung des Isobutyrates 2,2,4-Trimethyl-1 ,3-Pentandiol, **dadurch gekennzeichnet, dass** die Kondensationsreaktion des Isobutyraldehyds in Gegenwart von einer Mischung aus Natriumhydroxid und Natriumisobutyrate als Katalysator in einem Gewichtsverhältnis von 3:1 geführt wird, zuerst bei 58-60% Umwandlungsstufe von Isobutyraldehyd zu Isobutyrate 2,2,4-Trimethyl-1,3-Pentandiol, bei einer Temperatur von 58-62 °C während 8-12 Minuten und dann bei 68-72% Umwandlungsstufe von Isobutyraldehyd zu Isobutyrat 2,2,4-Trimethyl 1,3-Pentandiol bei einer Temperatur von 88-92 °C während 3-7 Minuten, wonach aus dem Gemisch Isobutyrat 3-Hydroxy-2,2,4-Trimethylpentil und Isobutyrat 3-Hydroxy-2,2-Dimethyl-1-(1-Methylethyl) Propyl wird wässrige Lösung des Katalysators durch Absetzen und Durchwaschen der organischen Phase nach der Kondensationsreaktion abgetrennt und der verbleibende Teil der Mischung nach der Kondensationsreaktion wird in vier Stufen durchgeführt so, dass im ersten Schritt wird die Destillation in einer Destillationskolonne mit drei Zonen mit struktureller Auffüllung geführt beim Atmosphärendruck, bei Kolonnenkopf- Temperatur von 61 °C und 100 °C am Kolonnenboden, in einem Gewichtsverhältnis des Rückflußes zum Destillat im Verhältnis von 0,5:1, und als Trennmittel wird Prozesswasser in einer Menge von 0, 1 % der Strömung des Gemisches in die erste Kolonne verwendet und am Kolonnekopf wird Isobutyraldehyd gesammelt, in der zweiten Stufe wird die Destillation in einer Destillationskolonne mit zwei strukturellen Ausfüllungen geführt, unter Vakuum 12 kPa abs. bei einer Kolonnenkopf- Temperatur von 49 ° C, und bei Kolonnenboden-Temperatur von 140 °C, in einem Gewichtsverhältnis des Rückflußes zum Destillat im Verhältnis von 3 1, und aus der bewässerten rohe Mischung von Estern, die ausgeschöpfte Flüssigkeit aus der ersten Kolonne bildet wird das Wasser abdestilliert, in der dritten Stufe wird die Destillation in einer Kolonne mit zwei Ausfüllungen geführt beim Vakuum 1,3 kPa abs. bei einer Kolonnenkopf-Temperatur von 121 °C und Kolonnenboden-Temperatur von 138°C, und ein Gewichtsverhältnis des Rückflußes zum Destillat im Verhältnis von 15:1, und am Kolonnenkopf wird die leichte Fraktion, die 2,2,4-Trimethylpentandiol-1, 3 enthält , und in der vierten Stufe wird die Destillation in einer Destillationskolonne mit zwei Ausfüllungen geführt, unter Vakuum 1,3 kPa abs., bei einer Kolonnenkopf-Temperatur von 126°C und Kolonnenboden-Temperatur von 145 ° C, in einem Gewichtsverhältnis des Rückflußes zum Destillat im Verhältnis von 1:1.

## Revendications

1. Procédé de fabrication d'un mélange d'hydroxyesters aliphatiques, en particulier d'aldéhyde isobutyrique qui consiste à faire condenser l'aldéhyde isobutyrique en présence d'un catalyseur basique partiellement récupéré du procédé, et ensuite à un dégagement par distillation de l'aldéhyde isobutyrique n'ayant pas réagi et à évaporer le 2,2,4-triméthyl-1,3-pentanodiol nonché à un dégagement par distillation du 2,2,4-triméthyl-1,3-pentanodiol isobutyrate se caractéristant par ce que la réaction de condensation de l'aldéhyde isobutyrique est réalisée en présence du mélange de l'hydroxide de sodium et de l'isobutyrate de sodium en tant que catalysateur en proportion pondérale 3:1, tout d'abord à 58-60 % de transformation de l'aldéhyde isobutyrique en 2,2,4-triméthyl-1,3-pentanodiol isobutyrate à la température 58-62°C pendant 8-12 minutes, et ensuite à 68-72 % de transformation de l'aldéhyde isobutyrique en 2,2,4 triméthyl-1,3-pentanodiol isobutyrate à la température 88-92°C pendant 3-7 minutes, ensuite du mélange du 3-hydroxy-2,2,4-triméthylpentyl isobutyrate et du 3-hydroxy-2,2-diméthyl-1-(1-méthyléthylo)propyl isobutyrate une solution aqueuse se dégage du catalysateur par le repos et le lavage de la phase organique du mélange après la réaction de la condensation, et le procédé de distillation de l'autre partie du mélange après la réaction de la condensation est effectué en quatre étapes de telle manière qu'à la première étape la distillation est effectuée dans une colonne avec trois sections de remplissage structurel sous pression atmosphérique, à la température du sommet de la colonne 61°C et la température en bas de la colonne 100°C, et proportion pondérale du flegme au distillat 0,5:1, et comme facteur séparant est utilisée l'eau de procédé en quantité de 0,1% au flux du mélange introduit à la première colonne, et à son sommet l'aldéhyde isobutyrique est reçu, à la deuxième étape la distillation est menée dans la colonne avec deux sections de remplissage structurel, sous vide de 12 kPa abs., à la température du sommet de la colonne 49°C et la température en bas de la colonne 140°C, et proportion pondérale du flegme au distillat 3:1, tandis que du mélange brut impregné d'eau contenant les esters constituant le liquide épuisé de la première colonne, est distillée l'eau, à la troisième étape la distillation est menée dans la colonne avec deux sections de remplissage, sous vide de 1,3 kPa abs., à la température du sommet de la colonne 121 °C et la température en bas de la colonne 138°C, et proportion pondérale du flegme au distillat 15:1, et au sommet de la colonne la fraction légère est reçue contenant 2,2,4-triméthyl pentanodiol-1,3, et à la quatrième étape la distillation est menée dans la colonne avec deux sections de remplissage sous vide de 1,3 kPa abs., à la température du sommet de la colonne 126°C et la température en bas de la colonne 145°C, et proportion pondérale du flegme au distillat 1:1.
